## Europäisches Patentamt

(19) **European Patent Office**

**Office européen des brevets**

(11) Veröffentlichungsnummer: **0 115 835**

**B1**

(12) **EUROPÄISCHE PATENTSCHRIFT**

(45) Veröffentlichungstag der Patentschrift:
**18.05.88**

(21) Anmeldenummer: **84100882.4**

(22) Anmeldetag: **27.01.84**

(51) Int. Cl.⁴: **A 61 M 1/14,** A 61 M 1/30,
A 61 M 1/34

(54) **Vorrichtung zur Entfernung von Wasser aus Blut.**

(30) Priorität: **28.01.83 DE 3302804**

(43) Veröffentlichungstag der Anmeldung:
**15.08.84 Patentblatt 84/33**

(45) Bekanntmachung des Hinweises auf die Patenterteilung:
**18.05.88 Patentblatt 88/20**

(84) Benannte Vertragsstaaten:
**AT BE CH DE FR GB IT LI LU NL SE**

(56) Entgegenhaltungen:
**EP-A-0 071 951
EP-A-0 088 900
DE-A-2 522 180
DE-A-2 605 181
DE-A-2 634 238
DE-A-2 636 290
DE-A-3 111 061
FR-A-2 398 507**

(73) Patentinhaber: **Fresenius AG, Gluckensteinweg 5,
D-6380 Bad Homburg (DE)**

(72) Erfinder: **Polaschegg, Hans- Dietrich, Dr.,
Grünwiesenweg 9, D-6370 Oberursel 4 (DE)**

(74) Vertreter: **Luderschmidt, Wolfgang, Dr. Dipl-
Chem., Görtz, Dr. Fuchs, Dr. Luderschmidt
Patentanwälte Sonnenberger Strasse 100
Postfach 26 26, D-6200 Wiesbaden (DE)**

## Beschreibung

Die Erfindung betrifft eine Vorrichtung zur Entfernung von Wasser aus Blut im extrakorporalen Kreislauf gemäß dem ersten Teil von Anspruch 1.

Im Bereich der Intensivmedizin führt die hochkalorische parenterale Ernährung häufig zur Überwässerung von Patienten und somit zum Blutdruckabfall, was ein Aussetzen der Filtrationsleistung der Niere zur Folge hat. Um diese zu entwässern, kann man daher die bereits bekannten und üblichen Geräte, wie die eingangs erwähnte Vorrichtung oder volumenkontrollierte Dialysemaschinen verwenden, die im allgemeinen zur reinen Dialyse eingesetzt werden, bei der neben dem Entzug von Wasser auch eine Reinigung des Bluts von Stoffwechselprodukten stattfindet.

Infolge dieser speziellen Einsatzweise sind diese bekannten Maschinen auf den speziellen Zweck zugeschnitten und relativ kompliziert aufgebaut, so daß sie für nicht trainiertes Personal praktisch nicht zu bedienen sind.

Beispiele für derartige, insbesondere eingangsseitig beschriebene Dialysevorrichtungen sind die bekannten Single-Needle-Dialysevorrichtungen, wie sie beispielsweise aus den Druckschriften US-A-3 811 800, 3 791 767 oder der DE-A-2 455 917 bekannt sind. Mit Hilfe derartiger Vorrichtungen wird Blut in einem geschlossenen Kreislauf intermittierend umgepumpt, wobei mit Hilfe der Blutpumpe im geschlossenen Kreislauf ein Überdruck erzeugt wird, der die Ultrafiltration von Plasmawasser im Hämofilter bewirkt.

Demgemäß wurde im medizinischen Bereich die arterio-venöse Hämofiltration als wesentliche Vereinfachung empfunden, weil sie ohne jedes Steuerungsgerät auskommt. Bei diesem Verfahren wird chirurgisch sowohl ein arterieller als auch ein venöser Zugang geschaffen und dazwischen ein extrakorporaler Kreislauf aufgebaut, der ein Hämofilter aufweist. Bei geschlossenem Kreislauf ergibt sich die Blutflußgeschwindigkeit aus dem Durchflußwiderstand des extrakorporalen Systems und aus dem arteriell-venösen Druckunterschied, während die Filtrationsleistung von den Filtrationseigenschaften des Filters und dem venösen Druck abhängt. Das System ist so aufgebaut, daß das Filtrat in einen Auffangbeutel abfließen und von Zeit zu Zeit die aufgefangene Menge festgestellt und kontrolliert werden kann.

Dieses System hat, obwohl es einfach ist, einige schwerwiegenden Nachteile:

Zunächst ist der extrakorporale Kreislauf in keiner Weise überwacht. Da ein arterieller Anschluß vorhanden ist, besteht somit eine akute Gefahr des Blutverlustes in die Umgebung, wenn ein Defekt im System auftritt oder sich ein Anschluß löst. Dies kann innerhalb weniger Minuten zum Verbluten des Patienten führen, falls der extrakorporale Kreislauf nicht rechtzeitig wieder geschlossen wird.

Weiterhin kann die Filtrationsleistung - abgesehen von der Wahl des eingesetzten Hämofilters und des Schlauchmaterials - nicht geregelt und kontrolliert werden. Dieser prinzipielle Nachteil führt dazu, daß die Filtrationsleistung niedrig ist, wenn die Überwässerung groß und der Blutdruck abgefallen ist, und sich erst mit zunehmender Entwässerung und steigendem Blutdruck wieder erhöht. Dementsprechend kann ein überwässerter Patient nur langsam aus einem infolge Überwässerung aufgetretenen Schock herausgeholt werden, jedoch um so schneller in einen infolge überhöhter Entwässerung erzeugten Schock fallen. Insofern verhält sich also die Filtrationsleistung bei der üblichen arterio-venösen Hämofiltration gerade entgegengesetzt zum wünschenswerten Zustand.

Aus DEUTSCHE MEDIZINISCHE WOCHENSCHRIFT, 102 (1977), S. 1804 - 1807 ist eine Hämofiltrationseinheit bekannt, bei der Blut einem Hämofilter zugeführt wird, an das auf der Filtratseite mittels einer Unterdruckpumpe Unterdruck angelegt wird. Das durch diesen Filter dem Blut entzogene Ultrafiltrat wird in einer Ultrafiltratmeßkammer aufgefangen, in der das Ultrafiltratvolumen bestimmt werden kann.

Eine derartige Vorrichtung ist jedoch nicht ausreichend sicher und muß dementsprechend häufig bei der Behandlung des Patienten überwacht werden. Zudem kann das Filter verstopfen, falls dem Blut zuviel Plasmawasser entzogen wird, mit der Folge, daß die gesamte Behandlung abgebrochen werden muß.

Weitere Hämofiltrationsvorrichtungen sind aus MEDIZIN-TECHNIK, 101 (1981), S. 124 - 128 sowie aus J. La. Clin. Med., 85 (1975), S. 373/374 bekannt. Auch bei diesen Hämofiltrationsvorrichtungen wird an das Hämofilter auf der Ultrafiltratseite Unterdruck angelegt und das entstandene Ultrafiltrat in einem Meßbehälter aufgefangen. Es findet hier ebenfalls keine wirksame Kontrolle der Ultrafiltration statt.

Weiterhin ist in der US-A-3 902 490 eine tragbare künstliche Niere beschrieben, bei der Dialysierflüssigkeit in einem geschlossenen Kreislauf durch den Dialysator geführt wird, wobei mit dieser Vorrichtung im wesentlichen kein Ultrafiltrat entzogen wird.

Desweiteren zeigt die DE-A-3 111 061 eine typische Hämodiafiltrationseinrichtung, bei der bilanziert - wie aus der Zeichnung ersichtlich ist - Ultrafiltrat dem Blutkreislauf entnommen wird und zugleich eine bestimmte Menge Substitutionsflüssigkeit stromab des Dialysefilters dem Blutkreislauf wieder zugeführt wird. Dabei ist die Differenz zwischen der abgezogenen Ultrafiltratsmenge und der zugeführten Substitutionsflüssigkeitsmenge die tatsächlich ultrafiltrierte, also dem Patienten entnommene Wassermenge.

Insofern ist in der letztgenannten Druckschrift eine kontinuierlich arbeitende Hämodialyseeinrichtung gezeigt, bei der der Blutkreislauf einerseits und der Ultrafiltratkreis andererseits nach einem speziellen Programm aufeinander abgestimmt sein müssen. Insofern sind grundsätzlich bestimmte Blutfußdaten bei bestimmten Ultrafiltrationsraten und Substitutionsraten zum wirksamen Betrieb einer derartigen Dialysevorrichtung notwendig. Nur dann kann gewährleistet werden, daß eine derarige Hämodiafiltrationsmaschine ohne Probleme arbeitet. Ihr Betrieb ist jedoch nur mit Hilfe hochspezialisierten Fachkräften in speziellen Dialyseabteilungen durchführbar, d. h. eine derartige Vorrichtung kann nicht von medizinischem Hilfspersonal ohne spezielles Training bedient werden.

Die DE-A-2 634 238 zeigt lediglich ein Bilanziersystem für eine Hämodialysemaschine, bei der in geschlossenem Kreislauf identische Volumina ausgetauscht und zugleich wieder zugeführt werden. Eine derartige Vorrichtung gewährleistet lediglich, daß gleiche Diafiltratmengen durch gleiche Mengen Substituat ersetzt werden, wie dies u. U. auch mit der Vorrichtung gemäß DE-A-3 111 061 angestrebt wird.

Demgegenüber liegt der Erfindung die Aufgabe zugrunde, die Vorrichtung der eingangs erwähnten Art derart fortzubilden, daß bei geringem technischen Aufwand eine sichere volumenkontrollierte Entwässerung von Blut möglich ist.

Diese Aufgabe wird durch die kennzeichnenden Merkmale des Anspruchs 1 gelöst.

Gegenüber der arterio-venösen Hämofiltration weist die erfindungsgemäße Vorrichtung zunächt den Vorteil auf, daß sie zweckmäßigerweise mit einem venösen Zugang betrieben wird, so daß in aller Regel nicht die Gefahr des Verblutens droht. Ein derartiger Zugang kann - wie bei der Dialyse üblich auf einfache Weise hergestellt werden, was gegenüber der arterio-venösen Hämofiltration einen weiteren Fortschritt darstellt, da bei dieser Methode komplizierte Femoralkatheter gesetzt werden müssen.

Weiterhin zwingt die erfindungsgemäße Vorrichtung diesem Filtrationsverfahren ihre Arbeitsweise auf, so daß die Filtrationsleistung und -rate ausschließlich von der erfindungsgemäßen Vorrichtung und praktisch nicht vom Blutkreislauf des zu behandelnden Patienten bestimmt wird, wie dies bei der arterio-venösen Hämofiltration der Fall ist. Demzufolge kann ein überwässerter Patient mit der erfindungsgemäßen Vorrichtung erheblich schneller entwässert werden, als dies bei der arterio-venösen Hämofiltration der Fall ist. Diese Unabhängigkeit von der Steuerung durch den Blutdruck des Patienten stellt eine wesentliche Verbesserung dieser Hämofiltrationsart dar, zumal gerade in kritischen Situationen die arterio-venöse Hämofiltration nur bedingt einsetzbar ist, da der Blutdruck zu schwach ist.

Die bei der erfindungsgemäßen Vorrichtung vorgesehenen Klemmen, die stromauf und stromab der Ultrafiltratmeßkammer vorgesehen sind, dienen sowohl zur Überwachung der gesamten Anordnung als auch zur Entleerung der Ultrafiltratmeßkammer, wobei diese Meßkammer mit einem Auffangbeutel verbunden ist. Die Klemmen arbeiten so, daß die Anzahl der Zyklen, die in dem Überwachungsgerät gespeichert wird, korreliert ist mit dem Volumen des erhaltenen Ultrafiltrats. Somit kann also durch diese Anzahl der Zyklen die gewünschte Ultrafiltratmenge eingestellt werden, die ebenfalls mit dem Volumen der Ultrafiltratmeßkammer korreliert ist.

In einer bevorzugten Ausführungsform sind die Volumina der Pumpkammer und der Ultrafiltratkammer so abgestimmt, daß pro Zyklus das Blutvolumen höchstens um die Hälfte durch Abpressen des Plasmawassers verringert wird. Hierdurch wird sichergestellt, daß nur eine bestimmte Höchstmenge Plasmawasser je Zyklus aus dem Blut entfernt wird.

Vorteilhafterweise ist jedoch das aus dem Blut entfernte Wasservolumen geringer und liegt etwa zwischen 25 und 33 %, bezogen auf das Gesamtblutvolumen/Zyklus. Bei einer derartigen Filtrationsleistung wird das Filter nicht verstopft und es kann der gefilterte Blutrestbestandteil ohne Schwierigkeiten zum Patienten zurückgefördert werden.

Mit der erfindungsgemäßen Vorrichtung kann nicht nur hämofiltriert werden; sie kann gemäß einer bevorzugten Ausführungsform zugleich exakt bilanzieren, so daß also gleiche Teile Infusionslösung und Filtrat dem Körper zu- bzw. abgeführt werden können.

Insgesamt gesehen ist die gesamte Vorrichtung gegenüber den komplizierten Dialysemaschinen sehr einfach aufgebaut und eignet sich somit für jede Intensivstation. So besteht beispielsweise das gesamte Schlauchsystem einschließlich der Pumpkammer und der Hämofiltratmeßkammer aus einem Einmalsystem, das steril geliefert wird und nach Gebrauch weggeworfen werden kann. Durch zweckmäßigerweise angebrachte Markierungen läßt sich dieses Einmalsystem auf einfache Weise den entsprechenden Bereichen der erfindungsgemäßen Vorrichtung auch von weniger geübtem Bedienungspersonal zuordnen, was für die Bedienungsfreundlichkeit von entscheidendem Vorteil ist.

Schließlich läßt sich die erfindungsgemäße Vorrichtung zweckmäßigerweise mit Hilfe eines Mikroprozessors überwachen, der auch kleine Blutlecks oder Apparatefehler, wie ein Verschluß eines Leitungsstücks oder des Filters, sofort erkennt und dann einen entsprechenden Alarm gibt bzw. die Vorrichtung abschaltet.

Weitere Einzelheiten, Vorteile und Ausführungsformen sind in der nachfolgenden Beschreibung mehrerer Ausführungsbeispiele unter Bezugnahme auf die Zeichnung erläutert.

Es zeigen

Fig. 1 schematisch eine erste Ausführungsform einer Vorrichtung zur Entfernung von Wasser aus Blut,

Fig. 2 einen Längsschnitt durch eine Blutpumpen- oder Ultrafiltratmeßkammer,

Fig. 3 eine Druck/Volumencharakteristik einer in Fig. 2 gezeigten Meßkammer,

Fig. 4 einen Längsschnitt durch eine Membranpumpe zum Einsatz in der in Fig. 1 gezeigten Anordnung und

Fig. 5 eine weitere schematische Darstellung einer weiteren Ausführungsform der Vorrichtung zur Entfernung von Wasser aus Blut.

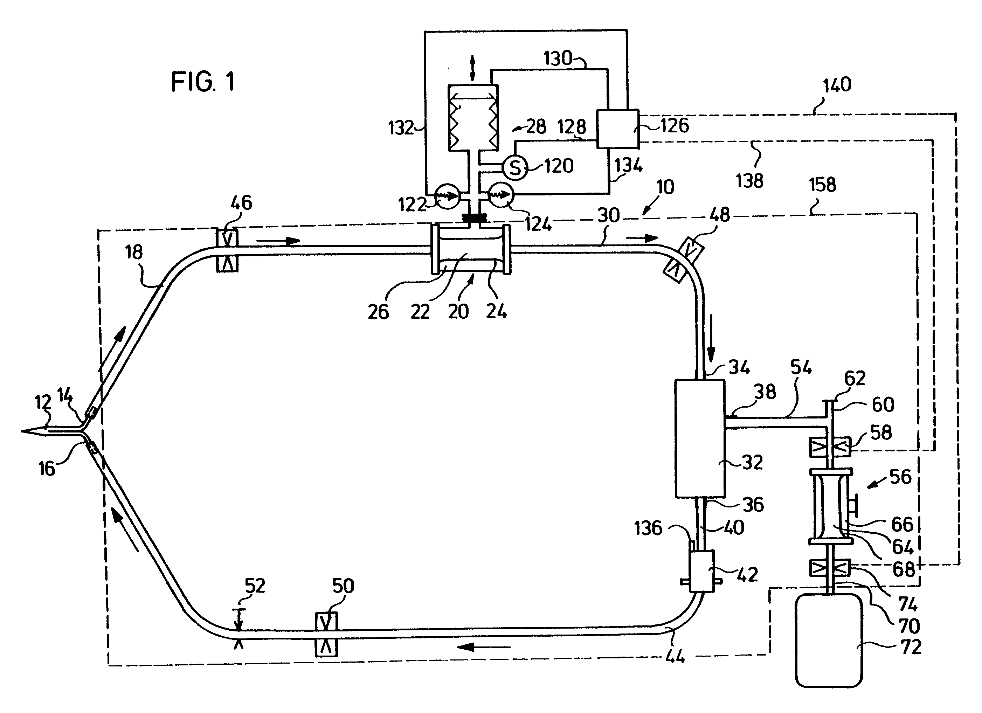

In Fig. 1 ist mit 10 eine Vorrichtung zur Entfernung von Wasser aus Blut in ihrer ersten Ausführungsform gezeigt. Diese Vorrichtung 10 weist zunächst eine Hohlkanüle 12 auf, mit der ein Blutanschluß an eine Vene hergestellt werden kann. Gemäß der in Fig. 1 gezeigten Ausführungsform ist diese Hohlkanüle 12 an ihrem anderen Ende y-förmig ausgebildet und bildet somit zwei Anschlusse 14 und 16, die mit entsprechenden Schlauchstücken verbunden werden können. Vorteilhafterweise ist sie als einfache Nadel ausgebildet, die sich dann entsprechend verzweigt, wobei der Vermischungseffekt praktisch zu vernachlässigen ist.

Der Anschluß 14 dieser Hohlkanüle 12 dient zur Ableitung von Blut und ist mit einem ersten Schlauchstück 18 verbunden. Das andere Ende dieses Schlauchstücks 18 ist mit der Pumpkammer 20 verbunden, die nachstehend eingehend erläutert wird. Diese Pumpkammer 20 weist vorteilhafterweise zwei voneinander geteilte Innenräume auf, nämlich den Kammerteil 22 und den von diesem Kammerteil 22 durch eine flexible Wand 24 abgetrennten Kammerteil 26. Dabei ist der Kammerteil 22 in Strömungsverbindung mit dem Schlauchstück 18, während der Kammerteil 26 mit einer Balgpumpe 28 verbunden ist.

Die Pumpkammer 20 ist stromab mit einem weiteren Schlauchstück 30 verbunden, wobei dieses Schlauchstück 30 wiederum in Strömungsverbindung mit dem Kammerteil 22 und dem Schlauchstück 18 ist. Das andere Ende dieses Schlauchstücks 30 mündet in einen Filter 32, der vorteilhafterweise als Hämofilter ausgebildet ist und infolgedessen drei Anschlüsse aufweist, nämlich den mit dem Schlauchstück 30 verbundenen Anschluß 34, den Auslaß 36 und den Filtratauslaß 38.

Dieses Filter ist vorteilhafterweise als Hämofilter ausgebildet, in dem überschüssiges Plasmawasser durch die im Filter vorgesehenen Membranen hindurchgedrückt und am Filtratauslaß 38 abgeschieden wird. Vorteilhafterweise weist dieses Filter 32 eine Vielzahl von membranartigen Hohlfäden auf, durch die das Blut hindurchgeleitet wird und an deren Membranwand das Filtrat abgepreßt wird.

Der Auslaß 36 ist mit dem Schlauchstück 40 verbunden, dessen anderes Ende mit einer Luftabscheidekammer 42 in Verbindung steht.

Die Luftabscheidekammer 42, in der aus Sicherheitsgründen die überschüssige Luft abgeschieden wird, ist an ihrem Bodenauslaß mit dem Schlauchstück 44 in Verbindung, das zum Anschluß 16 der Hohlkanüle 12 zurückgefuhrt ist.

Somit stellen das Schlauchstück 18, das Kammerteil 22 der Pumpkammer 20, das Schlauchstück 30, das Filter 32, das Schlauchstück 40, der Luftabscheider 42 und das Schlauchstück 44 einen geschlossenen Strömungskreis dar, in dem das Blut zirkulieren kann, d.h. ausgehend von einem Blutanschluß in einem gegenüber der Atmosphäre abgeschlossene Kreislauf zu einem weiteren Blutanschluß umgepumpt wird.

Da gemäß dieser Ausführungsform nur ein einziger Blutanschluß mittels der Hohlkanüle 12 hergestellt ist, kann das Zu- und Abpumpen von Blut üblicherweise nur intermittierend erfolgen, so daß entsprechende Einrichtungen zum Sperren der Zufuhr und Abfuhr von Blut im Schlauchsystem vorgesehen sein sollen.

Das Schlauchstück 18 kann durch eine Klemme 46 und das Schlauchstück 30 durch eine Klemme 48 geöffnet und geschlossen werden, wobei die Klemmen 46 und 48 im Gegentakt arbeiten.

Weiterhin kann das Schlauchstück 44 durch die Klemme 50 geöffnet und geschlossen werden. Zugleich ist stromab der Klemme 50 im Bereich des Schlauchstücks 44 ein Drosselorgan 52 vorgesehen, mit dem die Menge des Blutflusses durch das stromauf des Drosselorgans befindliche Schlauchsystem gesteuert werden kann.

Vorteilhafterweise wird die Klemme 50 analog zur Klemme 48 gesteuert, d.h. im Gegentakt zur Klemme 46.

Darüber hinaus läßt sich das Drosselorgan 52 - vorteilhafterweise manuell - so einregeln, daß der gewünschte Überdruck im stromauf befindlichen Leitungssystem erzeugt wird.

Der Filtratauslaß 38 des Filters 32 ist mit einem Schlauchstück 54 verbunden, dessen anderes Ende an eine Filtratmeßkammer 56 angeschlossen ist.

Zweckmäßigerweise läßt sich das Schlauchstück 54 durch eine Klemme 58 abklemmen und wieder öffnen, wobei die Arbeitsweise dieser Klemme 58 bei Normalbetrieb zweckmäßigerweise dem Takt der Klemmen 48 und 50 entspricht.

Das Schlauchstück 54 weist weiterhin einen Entlüftungsstutzen 60 auf, der mit einer bakteriensperrenden luftdurchlässigen Membran 62 verschlossen ist, die zweckmäßigerweise aus einer mikroporösen hydrophoben Membran aus PTFF u. dgl. besteht, wobei der Porendurchmesser unter 1 μm liegt.

Vorzugsweise ist die Filtratmeßkammer 56 der Pumpkammer 20 weitgehend ähnlich und weist insbesondere ein Kammerteil 64 und ein Kammerteil 66 auf, die durch eine flexible Wand 68 voneinander getrennt sind.

Der Kammerteil 64 ist dabei in Strömungsverbindung mit dem Schlauchstück 54 auf der einen Seite und dem Schlauchstück 70 auf der anderen Seite, dessen anderes Ende mit einem Auffangbeutel 72 verbunden ist. Somit besteht eine Flüssigkeitsverbindung zwischen dem Schlauchstück 54, dem Kammerteil 64, dem Schlauchstück 70 und dem Auffangbeutel 72, in dem letztlich das entwickelte Filtrat aufgefangen wird.

Vorteilhafterweise läßt sich das Schlauchstück 70 ebenfalls durch eine Klemme 74 absperren, die im Takt zur Klemme 46 und im Gegentakt zu dem Klemmen 48, 50 und 58 arbeitet.

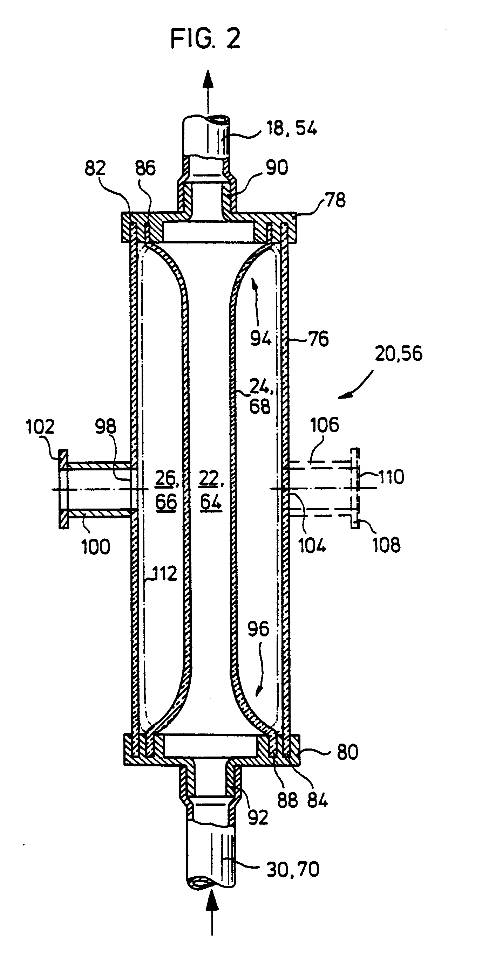

In Fig. 2 ist die erfindungsgemäße Pumpkammer 20 im Längsschnitt gezeigt, die in einer bevorzugten Ausführungsform in ihrer prinzipiellen Gestaltung der Filtratmeßkammer 56 im wesentlichen gleicht. Wie bereits vorstehend erläutert, weist die Kammer 20, 56, jeweils voneinander getrennte Kammerteile 22, 64 bzw. 26, 66 auf, die durch eine flexible Wand 24, 68, getrennt sind.

Die Kammer 20, 56 besteht im wesentlichen aus einem starren, vorteilhafterweise durchsichtigen Rohrstück 76, das an seinen beiden Enden durch Endplatten 78 und 80 geschlossen ist. In diesen Endplatten 78 und 80 sind jeweils ringförmige Nuten 82 und 84 vorgesehen, in die das Rohrstück 76 dicht haftend eingesetzt ist, so daß es einen geschlossenen Körper darstellt.

Die Endplatten 78 und 80 weisen jeweils eine weitere konzentrische, innenliegende ringförmige Nut 86 und 88 auf, in die die schlauchförmige, flexible Wand 24, 68 ebenfalls dicht haftend eingesetzt ist, so daß zwischen den Kammerteilen 26, 66 und 22, 64 keine Strömungsverbindung besteht.

Die Endplatten 78 und 80 sind darüber hinaus mit einem Flüssigkeitsdurchlaß in Form eines Stutzens 90 und 92 versehen, die mit den Schlauchstücken 18, 30 bzw. 54, 70 verbunden werden können, so daß die vor stehend eingehend erläuterte Strömungsverbindung erhalten wird.

Gemäß der in Fig. 2 gezeigten Ausführungsform ist die schlauchförmige Wand 24, 68 in den Endbereichen 94, 96 in gleicher Weise wie ein Gummischlauch quer gespannt und in den ringförmigen Nuten 86 und 88 befestigt. Dagegen ist sie im mittleren Bereich des Rohrstücks 76 allenfalls etwas in Längsrichtung, nicht jedoch in Querrichtung gespannt, damit sie nicht Falten oder dergleichen bildet.

Diese Art der Schlauchanbringung ist zwar bevorzugt, nicht jedoch kritisch. Dementsprechend können auch andere Schlauchanbringungsmethoden verwandt werden. So kann beispielsweise der Schlauch auch ohne Querspannung und nur mit geringer Längsspannung oder sogar spannungsfrei in das Rohrstück 76 eingesetzt werden.

Wie bereits vorstehend erwähnt, wird das Rohrstück 76 zweckmäßigerweise starr gewählt, d.h. es gibt bei den eingesetzten Unter- oder Überdrücken praktisch nicht nach. Um auf die flexible, vorteilhafterweise schlauchförmige Wand 24 einen Unter- bzw. Überdruck auszuüben bzw. grundsätzlich druckentlastet im ringförmig abgeschlossenen Raum des Kammerteils 26, 66 zu arbeiten, ist das Rohrstück 76 wenigstens von einer Öffnung 98 durchsetzt, an die sich nach außen hin ein Stutzen 100 mit einem Flansch 102 anschließt.

Sofern die Kammer 26, 66 mit jeweils einem Auslaß und einem Einlaß versehen werden muß, ist eine weitere Öffnung 104 und ein weiterer Stützen 106 mit einem Flansch 108 vorgesehen, was in Fig. 2 gestrichelt gezeigt ist. Dabei kann der Flansch 108 mit einem bakteriendichten Filter 110 abgedeckt sein, gestattet also einen Druckausgleich des Kammerteils 26, 66. Dieses Filter 110 gleicht der vorstehend erwähnten mikroporösen Membran 62, die im übrigen vorteilhafterweise erst bei einem Überdruck von etwa 1 bar Wasser durchläßt.

Wie in Fig. 2 mit 112 gezeigt, läßt sich die schlauchförmige Wand 24, 68 bei Anlegen von Unterdruck über den Stutzen 100 derart expandieren, daß sie sich direkt an die Innenwand des Rohrstücks 76 anlegen kann. Andererseits geht sie infolge seiner Rückstellkraft in den Ausgangszustand, wie in Fig. 2 gezeigt, wieder zurück und kann bei entsprechend ausgeübtem Überdruck, was wiederum über den Stutzen 100 erfolgen kann, auch vollständig zusammengepreßt werden. Somit kann also der Innenraum des Rohrstücks 76 praktisch vollständig gefüllt und wieder entleert werden.

Als Material für den als Trennwand 24, 68 eingesetzten Schlauch werden hochelastische Materialien eingesetzt, beispielsweise organische Polymere und deren Gemische, wie Polyurethane, Kautschuke, Siliconkautschuke, Latex, Gummi, regenerierter Gummi u.dgl. Vorzugsweise wird Latex oder ein Siliconkautschuk unter der Bezeichnung Silastic® eingesetzt, die ggf. übliche Zusatz- und die Elastizität verbessernde Mittel aufweisen. Vorteilhafterweise läßt sich die Wand dieses Schlauchs derart erweitern, daß das Innenvolumen, bezogen auf den nicht gespannten Zustand des Schlauchs, wenigstens um das Fünffache, insbesondere um das Zehnfache vergrößert werden kann.

Dementsprechend kann der Schlauch gemäß einer bevorzugten Ausführungsform bis zum Zehnfachen seines Innenvolumens zusätzlich aufnehmen.

Die Wandstärke eines derartigen Schlauchs ist im wesentlichen nicht kritisch und liegt in einem Bereich von etwa 0,05 - 0,5, vorzugsweise 0,1 - 0,4 mm. Die Wandstärke und das Schlauchmaterial werden in Abhängigkeit von der gewünschten Druckcharakteristik und der Volumenerweiterung gewählt. So ist die Wandstärke des Schlauchs um so dicker, je höher der auf zuwendende Anfangsdruck ist.

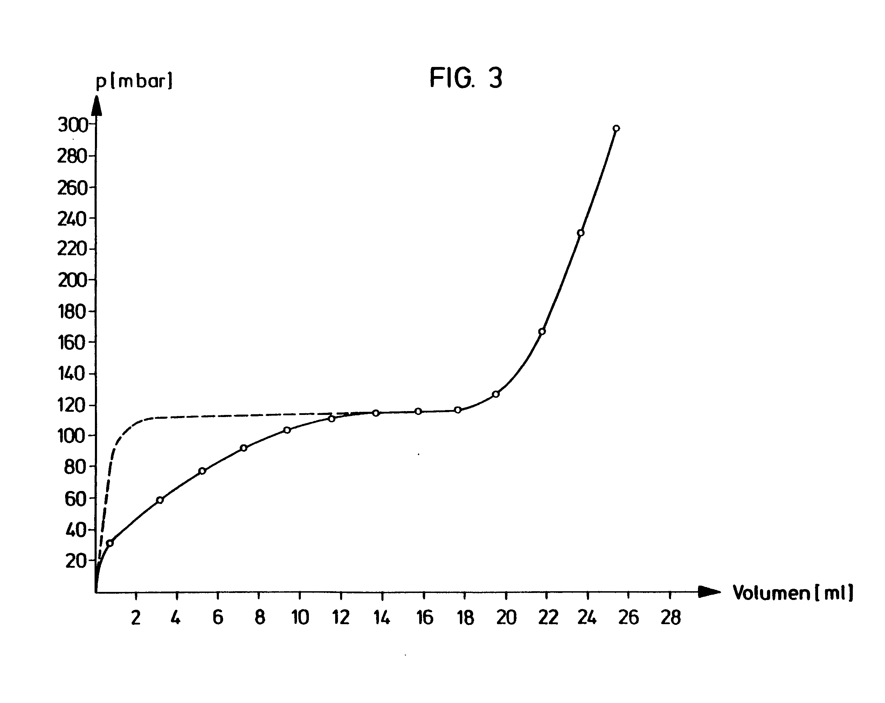

Gemäß einer bevorzugten Ausführungsform wird ein derartiger Schlauch gewählt, der in seiner Wandstärke von dem einen Ende zum anderen Ende hin abnimmt, wie dies beispielsweise bereits in Fig. 2 gezeigt ist. Demzufolge bläht sich der Schlauch zunächst an dem Ende auf, an dem die geringste Wandstärke vorliegt. Die Druckcharakteristik eines derartigen einsetzbaren Schlauches ist beispielsweise in Fig. 3 gestrichelt gezeigt. Demzufolge muß zunächst ein erheblicher Unterdruck angewandt werden, um das Volumen des Schlauches um etwa 1 - 2 ml zu vergrößern. Dieser Unterdruck wächst dann nur noch geringfügig an, bis sich das Volumen des Schlauchs bei etwa 20 - 22 ml befindet. Hier ist dann auch die vollständige Füllung des Schlauchs erreicht, so daß bei Anlegen eines größeren Unterdrucks praktisch keine Vergrößerung des Innenvolumens des Schlauchs mehr erreicht werden kann. Demzufolge liegt also der Schlauch, wie in Fig. 2 bei 112 gezeigt, an der Innenwand des Rohrstücks 76 an.

Ein solcher, vorstehend erwähnter Schlauch, dessen Wandstärke über die Gesamtlänge der Kammer um bis zu 50 % abnimmt, kann beispielsweise dadurch erzeugt werden, daß man einen Stab in eine flüssige polymere Masse zur Herstellung eines solchen Schlauchs, beispielsweise Latex, eintaucht und den Stab anschließend aus der flüssigen Masse herauszieht. Infolge des flüssigen Zustandes der Masse fließt ein Teil der Masse am Stab ab, so daß die Wandstärke des dadurch hergestellten Schlauchs von einem Schlauchende zum anderen Schlauchende stetig zunimmt.

Das Schlauchvolumen und die Länge des Schlauchs richten sich nach dem Einsatzzweck sowie der Auslegung der Vorrichtung. Üblicherweise wird der Durchmesser eines Schlauchs in einem Bereich von 5 - 15, vorzugsweise etwa 8 mm liegen, während die Länge des Schlauchs 5 - 20 cm betragen kann. Es sind jedoch aber auch kürzere oder längere Abmessungen denkbar, sofern dies der Einsatzzweck erfordert. Wenn nur geringe Blutvolumina befördert werden, wird man einen entsprechend geringer dimensionierten Schlauch einsetzen als bei größeren Blutumsätzen.

Wie bereits vorstehend erläutert, hängt jedoch das Volumen der in den Kammern 20, 56 umgepumpten Flüssigkeiten, nämlich Blut und Filtrat, von dem Innenvolumen des Rohrstücks 76 ab, das durch die Endplatten 78, 80 begrenzt ist. Das aus den Kammerteilen 22 und 26 gebildete Volumen $V_1$ der Kammer 20 und das aus den Kammerteilen 64, 66 gebildete Innenvolumen $V_2$ der Kammer 56 bestimmen durch ihr Verhältnis den Anteil des Ultrafiltrats an der gesamt durchgepumpten Blutmenge. Dieses Verhältnis kann bei überwässerten Patienten 3 : 1 betragen, d.h. bei einer Blutpumpenkammer 20 von 30 ml Inhalt kann die Ultrafiltrationsmeßkammer 56 einen Inhalt von 10 ml haben. Vorzugsweise wird daher das Verhältnis von $V_1 : V_2$ in einem Bereich von 5 : 1 bis 2 : 1 gehalten.

Dementsprechend kann das Volumen $V_1$ bzw. $V_2$ bis zu 80 bzw. 40 ml betragen, während der elastische Schlauch im drucklosen Zustand etwa 5 - 15 ml faßt.

Hinzuzufügen ist, daß als Material für den elastischen Schlauch alle wasserundurchlässigen Materialien in Frage kommen, die eine entsprechend große Bruchdehnung ( > 200 %) aufweisen, wozu die vorstehend genannten Materialien gehören.

Als Material für das Rohrstück 76 kommen Materialien in Frage, die bei entsprechender Dimensionierung druckbeständig und im wesentlichen starr sind. Hierzu gehören beispielsweise polymere Materialien, wie Polyethylen, Acrylglas, PVC u.dgl.

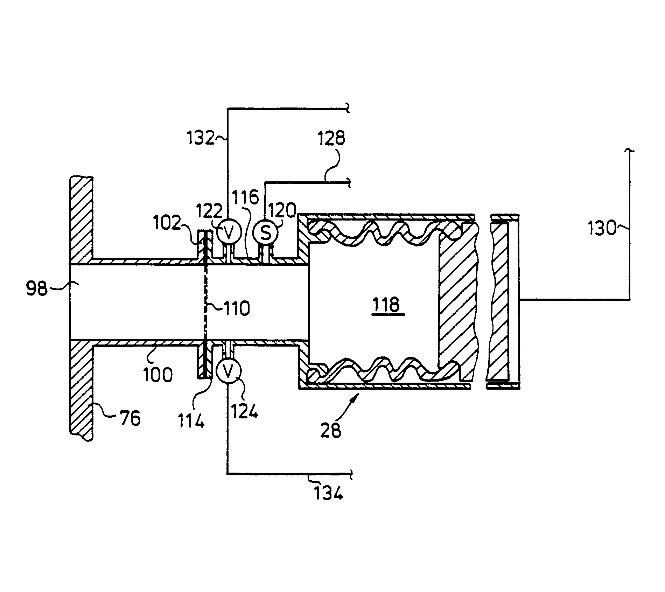

Gemäß der in Fig. 1 gezeigten Ausführungsform ist die Pumpkammer 20 mit einer Balgpumpe 28 verbunden. Der Anschluß sowie die Balgpumpe 28 selbst sind ausführlicher in Fig. 4 dargestellt. Dabei ist der Flansch 102 des Stutzens 100 mit einem entsprechend passenden Flansch 114 der Balgpumpe 28 verbunden.

Unter einer Balgpumpe 28 wird eine Pumpe mit konstantem Hubvolumen verstanden. Vorzugsweise wird eine Balgpumpe eingesetzt, deren Hubvolumen größer ist als das Innenvolumen des Rohrstücks 76 der Pumpkammer 20. Besonders bevorzugt ist eine Balgpumpe, deren Hubvolumen mehr als 20 % größer ist als das Speichervolumen der Pumpkammer 20.

Mittels der Balgpumpe 28 wird Luft aus dem Kammerteil 26 durch die Öffnung 98, den Stutzen 100 und das sich an den Flansch 114 anschließende Rohrstück 116 in den Balg 118 abgesaugt.

Diese Pumpe 28 kann sowohl druck- als auch zwangsgesteuert betrieben werden.

Bei der druckgesteuerten Ausführungsform erfolgt ein Umschalten der Pumpe, die natürlich nicht notwendigerweise als Balgpumpe ausgeführt sein muß, bei einem bestimmten oberen und unteren Druckwert innerhalb des Kammerteils 26. Dabei kann die Pumpe bei blutgefülltem Kammerteil 22 entweder abgeschaltet werden, wobei über ein entsprechendes, nicht gezeigtes Entlüftungsventil Luft zum Kammerteil 26 zutreten und sich die elastische, schlauchförmige Wand 24 in ihren Ausgangszustand unter Abpumpen von Blut begeben kann, oder in die Abpumpstellung umgeschaltet werden, wobei durch Zuführung von Luft mittels der Pumpe 28 die elastische Wand 26 ebenfalls wiederum entspannt werden kann.

Zur Bestimmung eines bestimmten Unterdrucks in dem Pumpsystem, insbesondere im Kammerteil 26 oder den damit in Verbindung stehenden Stutzen 100 und Rohrstück 116, ist ein Drucksensor 120 vorgesehen, mit dem die Pumpe bei einem vorher festgelegten Unter- und Überdruck umgeschaltet werden kann.

Bei der Zwangssteuerung, bei der die Balgpumpe 28 zwischen einem oberen und unteren Totpunkt, d.h. einem Höchst- und Tiefstdruckwert pendelt, ist ein derartiger Drucksensor 120 nicht zwangsläufig notwendig, wird jedoch vorteilhafterweise zur Überprüfung des Systems eingesetzt. Zweckmäßigerweise wird jedoch der Unter- und Überdruck in dem Kammerteil 26 bzw. der gesamten Pumpkammer 20 mit einem Unterdruckventil 122 und einem Überdruckventil 124 gesteuert. Diese Druckventile 122 und 124 öffnen sich selbsttätig, wenn ein bestimmter Druckwert erreicht ist. So wird man vorzugsweise das Unterdruckventil 122 so einstellen, daß dieses umschaltet, wenn sich die schlauchförmige elastische Wand 24 vollständig mit Blut angefüllt hat und sich bei 112 an das Rohrstück 76 angelegt hat.

Infolge der erläuterten bevorzugten Überdimensionierung der Balgpumpe 28 gegenüber der Pumpkammer 20 bewegt sie sich noch bis zum oberen Totpunkt weiter und schaltet erst am oberen Totpunkt um. Mit der Umschaltung am oberen Totpunkt erfolgt dann die Abförderung von Blut aus der Pumpkammer 20, und zwar dergestalt, daß der Kammerteil 22 vor dem Erreichen des unteren Totpunkts entleert ist. Zur Steuerung des dabei entstandenen Überdrucks ist das vorstehend erwähnte Überdruckventil 124 vorgesehen, das sich öffnet, wenn der Kammerteil 22 entleert ist.

Ein System, das eine ähnliche Pumpkammer und eine ähnliche Balgpumpe in einen Single-Needle-System aufweist, ist beispielsweise in der DE-A-3 205 449 beschrieben.

Die Arbeitsweise der Vorrichtung 10 gemäß Fig. 1 wird nachstehend beschrieben.

Beim Normalablauf des Filtratprogramms werden die Klemmen 46 und 74 geöffnet, während die Klemmen 48, 50 und 58 geschlossen bleiben. Die Balgpumpe 28 befindet sich in ihrem unteren Totpunkt und wird zur Einleitung des Saugbetriebs gestartet. Dabei wird, als unterer Totpunkt, der sich beim zwangsgesteuerten Betrieb automatisch ergibt, auch der Schaltpunkt der Pumpe bezeichnet, bei der im druckgesteuerten Betrieb der Umschaltvorgang auf eine andere Betriebsart erfolgt.

Die Balgpumpe 28 saugt nunmehr Blut über die Hohlnadel 12 und das Leitungsstück 18 in den Kammerteil 22, da der im Kammerteil 26 durch die Expansion des Balges 118 erzeugte Unterdruck die schlauchförmige flexible Wand 24 erweitert. Wenn sich diese schlauchförmige flexible Wand an die Innenoberfläche des Rohrstücks 76 angelegt hat, wird entweder das Unterdruckventil 122 geöffnet oder aber der Drucksensor 120 schaltet entsprechend die Balgpumpe 28 um. Ist dann der obere Totpunkt erreicht und die Balgpumpe 28 umgeschaltet, werden die Klemmen 46 und 74 geschlossen und die Klemmen 48, 50 und 58 geöffnet.

Durch die Kompression des Balges 118 wird auf den mit Blut gefüllten Kammerteil 22 ein Überdruck ausgeübt, der das Blut beschleunigt durch den Rückführungskreis zur Hohlnadel 12 zurückführen würde, sofern nicht ein Strömungswiderstand in Form des Drosselorgans 52 vorgesehen ist. Dieses Drosselorgan 52 erzeugt somit in dem sich an die Pump-Kammer 20 anschließenden Kreis einen definierten Überdruck, der eine definierte Ultrafiltration im Filter 32 bewirkt. Durch entsprechende Steuerung des Überdrucks bzw. der Kompressionsdauer, mit der die Balgpumpe 28 betrieben wird, wird die Filtratmeßkammer 56 unter Expandierung der flexiblen Wand 68 gefüllt. Dabei wird die in dem Kammerteil 66 vorliegende Luft durch das Filter 110 im wesentlichen drucklos entfernt, so daß sich in diesem Kammerteil kein Gegendruck aufbauen kann.

Im zwangsgesteuerten Zustand ist der Kammerteil 22 vor Erreichen des unteren Totpunkts von Blut entleert, so daß der Überdruck dann schlagartig ansteigt, was das öffnen des Überdruckventils 124 bewirkt. Im druckgesteuerten Zustand bewirkt dies einen Schaltvorgang am Sensor 120. Hierauf wird wieder die Blutpumpe 28 auf Saugbetrieb - wie vorstehend erläutert - umgeschaltet, wobei die Klemmen 46, 48, 50, 58 und 74 entsprechend umgesteuert werden.

Mit dem Öffnen der Klemme 74 wird die Ultrafiltratmeßkammer 56 infolge der Rückstellkraft des elastischen Schlauchs und des tieferhängenden Beutels 72 von Ultrafiltrat entleert, das in diesen Beutel 72 abfließt und von diesem aufgefangen wird.

Die Ultrafiltrationsrate wird dabei durch den Blutfluß, d.h. durch die Blutpumpengeschwindigkeit gesteuert. Diese hängt natürlich von der Ergiebigkeit des Shunts ab und läßt sich natürlich nur so weit erhöhen, als dies der Blutzugang, beispielsweise ein Shaldon-Katheter, zuläßt.

Um das durch das Volumenverhältnis Pumpkammer 20 Ultrafiltrationskammer 56 vorgegebene Ultrafiltrationsverhältnis sicherzustellen, muß überwacht werden, ob sich die Pumpkammer 20 tatsächlich auch bei jedem Hub der Balgpumpe 28 füllt und anschließend entleert. Es ist nämlich durchaus möglich, daß aufgrund einer mangelnden Ergiebigkeit des Blutanschlusses die Pumpkammer 20 nicht im vorgegebenen Zeitintervall gefüllt wird. Es ist weiterhin denkbar, daß entweder das Schlauchstück 18 abgeknickt wird oder aber eines der Schlauchstücke 30, 40 oder 44 abgeknickt wird oder das Filter 32 blockiert, z. B. weil es zu einer Gerinnung des Blutes kommt.

Zu diesem Zweck ist der Drucksensor mit einem Überwachungsgerät 126 über die Leitung 128 gekoppelt, das über die Leitung 130 von der Balgpumpe 28 jeweils am Totpunkt Steuersignale erhält.

Die nachfolgende Erläuterung geht davon aus, daß die Balgpumpe 28, wie dies in der bevorzugten Ausführungsform der Fall ist, zwangsgesteuert betrieben wird.

Zu Beginn des Überwachungsvorgangs sei die Balgpumpe 28 auf den Wert $p_1$ komprimiert und der darin aufgebaute Druck sei durch das Überdruckventil 124 eingestellt. Dieser Druck $p_1$ wird mit Hilfe eines in dem Überwachungsgerät 126 vorgesehen Mikroprozessors gespeichert.

Durch die Expansion der Balgpumpe 28 sinkt der Druck in der Pumpkammer stetig ab, wobei Blut angesaugt wird. Nach Erreichen der Zeit $t_1$ ist die Pumpkammer völlig expandiert, so daß das Unterdruckventil 122 bei dem Unterdruck $p_2$ aufmacht.

Die Betätigung von Überdruck- und Unterdruckventil 122 und 124 wird dabei über die Leitung 132 und 134 ebenfalls an das Überwachungsgerät 126 gemeldet.

Da der Balg 118 am Öffnungspunkt des Unterdruckventils 122 noch nicht an seinem Totpunkt angelangt ist, wird der Druck $p_2$ bis zur Erreichung dieses Punkts konstant gehalten.

Nach Erreichen des oberen Totpunkts wird wiederum ein entsprechendes Steuersignal an das Überwachungsgerät 126 abgegeben, das dann die zeit $t_2$ mißt, innerhalb derer der Balg 118 solange komprimiert wird, bis das Überdruckventil 124 aufmacht und ein entsprechendes Signal an das Überwachungsgerät 126 abgibt dabei wird zusätzlich noch die Gesamtzykluszeit $t_3$ gemessen.

Aus dem Druckverlauf und dem verhältnis $t_1 : t_3$, d.h. dem Verhältnis der effektiven Ansaugzeit zur Zykluszeit des Systems, kann abgelesen werden, ob das System blockiert.

Ist nämlich das Schlauchstück 18 abgeknickt, so kann kein Blut abgesaugt werden und es kann zunächst die Pumpkammer 20 nicht expandieren.

Als Folge davon wird der Unterdruck am Druckmonitor 120 sehr viel rascher abfallen und das Unterdruckventil 122 noch vor Erreichen der Zeit $t_1$ öffnen.

Ähnliche Verhältnisse ergeben sich beim Komprimieren des Balgs 118. Auch hier wird sich bei ordnungsgemäßem Betriebszustand ein bestimmtes Verhältnis von Kompressionszeit $t_2$ zur Zykluszeit $t_3$ einstellen, das stark verkürzt wird, wenn die Pumpleitung unterbrochen oder das Filter 32 zugesetzt ist.

Demzufolge kann auch mit dem Überwachungsgerät 126 das Auftreten von kleinen Lecks im System, die zu einem Blutverlust in die Umgebung führen, erkannt werden. Dieser Übertritt in die Umgebung ist beispielsweise aus der Pumpkammer 20 erschwert, da - wie in Fig. 4 gezeigt - ein hydrophobes Filter 110 zwischen den Flanschen 102 und 114 vorgesehen ist. das einerseits luftdurchlässig ist, andererseits jedoch nicht Blut oder Wasser durchläßt.

Vorteilhafterweise läßt sich mit dem Überwachungsgerät 126 auch ein Programm durchführen, das die Inbetriebnahmephase steuert. Während dieser Phase, beispielsweise innerhalb von 5 Minuten. muß das Gerät vom Anwender überwacht und ggf. eingestellt werden. So kann beispielsweise die Geschwindigkeit der Pumpe 28 oder der Drosselungsgrad mit dem Drosselorgan 52 reguliert werden. Am Schluß der Inbetriebnahmephase quittiert der Anwender, daß alles in Ordnung ist. Von nun an registriert der Mikroprozessor den als richtig bezeichneten Druckablauf. Weicht der Druck gegenüber dem Anfangswert entsprechend stark aß, wird Alarm gegeben, wobei sämtlichhe Funktionen eingestellt und sämtliche Klemmen geschlossen werden.

Wie bereits vorstehend erläutert, ist in dem extrakorporalen System ein Luftabscheider 42 vorgesehen, um der Gefahr einer Luftembolie vorzubeugen. Eine solche Luftabscheidekammer ist beispielsweise aus der DE-A-3 115 299 bekannt. Da in dem Luftabscheider 42 üblicherweise Überdruck vorliegt, kommt es, sofern kein Alarmfall vorliegt, nicht zu einen Leerlaufen der Kammer, sofern die in der Kammer vom Blut abgetrennte Luft durch das hydrophobe Filter 136 entfernt wird. Dieses hydrophobe Filter ist bei den vorliegenden Überdrücken gegen ein Durchtreten von Blut oder Plasma gesichert, da eine hydrophobe mikroporöse Membran aus PTFE mit Porengrößen unterhalb 1 μm und einem Wassereintrittsdruck von mehr als 1 bar eingesetzt wird.

Vorteilhafterweise wird das gesamte System mit Ausnahme der von diesem System trennbaren Balgpumpe 28 als Disposable-System in einem Stück steril bereitgestellt, wobei insbesondere die einzelnen Schlauchsegmente farbig markiert sind, um ein Einlegen in entsprechend farbig markierte Bereiche am Gerät zu erleichtern. Der Bedienungsaufwand erstreckt sich dann nur noch auf das Einlegen der Schlauchstücke 18, 30, 44, 54, 70 in die Klemmen 46, 48, 50, das Drosselorgan 52, die Klemme 58 und die Klemme 74, das Einstecken der Leitungen 30 und 40 in den Hämofilter 32 sowie das Einstecken der Leitungen 40 und 44 in den Luftabscheider 42 und den Anschluß der Balgpumpe 28 an die Pumpkammer 20.

Das System muß vor Beginn der Behandlung mit physiologischer Kochsalzlösung gefüllt werden. Es verfügt daher vorteilhafterweise über ein Füllprogramm.

Während des Füllprogramms bleiben die Klemmen 58 und 74 geschlossen, während die Klemmen 46, 48, 50 und das Drosselorgan 52 geöffnet sind.

An die y-förmige Hohlkanüle 12 wird ein Beutel mit Kochsalzlösung angehängt und die Balgpumpe beginnt zu pumpen. Sie saugt nun eine Zeitlang ein Luft-Flüssigkeits-Gemisch an, wobei das Gemisch auch immer in den Kochsalzbeutel zurückgefüllt wird. Ein Teil dieses Gemischs wird im Filter 32 abfiltriert und füllt somit die Leitung 54 bis zur Klemme 58, wobei die in der Leitung und im Filter 32 vorliegende Luft über die Membran 62 vorteilhafterweise entfernt wird. Diese Membran 62 kann aber auch entfallen, wenn man einen kleinen Fehler bei der Ultrafiltrationsratenbestimmung in Kauf nehmen will.

Die im System enthaltene Luft wird im Luftabscheider 42 von der Flüssigkeit getrennt und aus dieser, wie vorstehend erwähnt, entfernt. Durch diesen speziell gesteuerten Füllvorgang wird verhindert, daß der Auffangbeutel 72 mit Luft vollgepumpt wird.

Am Schluß der Behandlung muß das im System vorhandene Blut dem Patienten zurückgegeben werden. Dazu muß, wie sonst auch üblich, das Leitungsstück 18 von der Hohlkanüle 12 getrennt werden, wobei der Anschluß 14 geschlossen wird. Dieses Leitungsstück 18 wird wieder an einen Kochsalzbeutel angehängt und der Pumpbetrieb wird wieder aufgenommen, wobei das Drosselorgan 52 vollständig geöffnet wird, um eine Ultrafiltration weitgehend zu verhindern. Zugleich werden die Klemmen 58 und 70 wieder geschlossen. Demgemäß sind das Start- und Entleerungsprogramm praktisch identisch.

In einer weiteren Ausführungsform der in Fig. 1 gezeigten Vorrichtung 10 kann auch der Luftabscheider 42 wegfallen, wenn sichergestellt ist, daß im gesamten extrakorporalen Kreislauf kein künstlich erzeugter Unterdruck auftritt. Hierzu muß das Unterdruckventil 122 so eingestellt sein, daß es schon mit Sicherheit bei + 10 mm Hg öffnet. Weiterhin muß natürlich die elastische Wand 24 so eingestellt sein, daß es schon durch den relativ geringen venösen Blutdruck des Patienten voll aufgepumpt wird. Um dies zu kontrollieren, ist entweder eine optische Kontrolle am Beginn der Behandlung notwendig oder aber eine automatische Kontrolle der Füllung des elastischen Ausdehnungsgefäßes vorzunehmen, wie dies in den Druckschriften DE-A-3 205 449 und DE-A-3 131 075 erwähnt ist.

Zu erwähnen ist weiterhin, daß das Überwachungsgerät 126 auch automatisch die Zahl der Zyklen speichern kann, mit der die Pumpe 28 betrieben wird. Durch entsprechende Vorwahl kann die ultrafiltrierte Menge bestimmt und somit eine definierte Entwässerung des Patienten durchgeführt werden.

Eine solche Vorwahl ist im übrigen auch bei der arteriovenösen Ultrafiltration möglich. In dieser Ausführungsform sind die Leitung 18 mit der Arterie und die Leitung 44 direkt mit der Vene verbunden, während der Filtratkreislauf, wie in Fig. 1 gezeigt. gleich bleibt. Lediglich die Klemmen 58 und 74 sind mit dem Überwachungsgerät 126 über die Leitungen 138 und 140 verbunden, das die Zykluszahl entsprechend speichert und die Klemmen nach Erreichen der vorbestimmten Zyklen abstellt. Eine solche Überwachung mit dem Überwachungsgerät 126 muß nicht notwendigerweise vorgesehen sein.

In seiner weiteren Ausführungsform dieser arterio-venösen Hämofiltration sind die Klemmen 58 und 74 durch eine Nocke mechanisch angetrieben, wobei die Schließ- und Öffnungszyklen wieder entsprechend registriert werden können. Durch eine entsprechende Wahl der Zyklen kann somit die gewünschte abzufiltrierende Ultrafiltratmenge vorbestimmt werden.

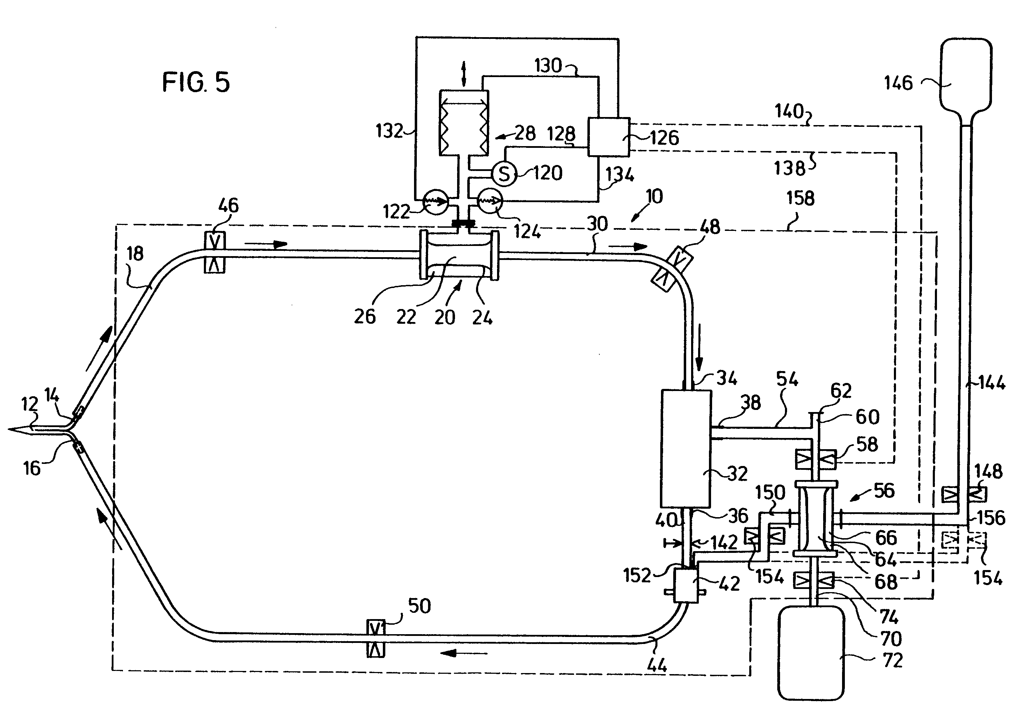

In Fig. 5 ist eine weitere Ausführungsform der erfindungsgemäßen Vorrichtung gezeigt, die im wesentlichen auf der Ausführungsform gemäß Fig. 1 aufbaut. Sie gestattet jedoch im Gegensatz zu der in Fig. 1 gezeigten Ausführungsform die Bilanzierung von Filtrat und zuzuführender Substitutionslösung.

Wie eingangs erläutert, ist die starke Überwässerung von Patienten durch die Gabe von hochkalorischen parenteralen Substitutionslösungen bedingt. Dabei entwässert die Vorrichtung gem. Fig. 1 bereits hochüberwässerte Patienten, ohne jedoch genau die Zugabe von Substitutionslösungen bilanzieren zu können.

Die in Fig. 5 gezeigte Ausführungsform schafft daher Abhilfe und bilanziert exakt die Zugabe von Substitutionslösungen.

In der in Fig. 5 gezeigten Ausführungsform wird lediglich schematisch die Änderung gegenüber der Ausführungsform gem. Fig. 1 gezeigt. Dabei ist die Drossel 52, die stromab des Luftabscheiders 42 gem. Fig 1 vorgesehen ist, durch eine Drossel 142 ersetzt, die stromauf des Luftabscheiders die Leitung 40 teilweise sperrt. Die gem. Fig. 3 gezeigte Ultrafiltratmeßkammer ist über den Stutzen 100 und eine Leitung 144 mit dem Infusionsbeutel 146 in Verbindung. Diese Leitung 144 kann durch eine Klemme 148 abgeklemmt und geöffnet werden.

Weiterhin ist der zweite Stutzen 106 über ein Leitungsstück 150 mit dem Infusionsstutzen 152, über den auch Heparin zugesetzt werden kann, mit dem Luftabscheider 42 verbunden. Dieses Leitungsstück 150 kann durch die Klemme 154 geschlossen werden.

Sofern die Ultrafiltratmeßkammer 56 nur einem Stutzen, beispielsweise 100, aufweist, verzweigt sich das Leitungsstück 144 bei 156, wobei an dieser Stelle das Leitungsstück 150 angesteckt ist. Dies ist in Fig. 5 gestrichelt dargestellt.

Die in Fig. 5 dargestellte Ausführungsform arbeitet folgendermaßen:

Bei Normalbetrieb ist keine Luft mehr in der Ultrafiltratmeßkammer 56 vorhanden, d.h. daß im Kammerteil 64 Ultrafiltrat und im Kammerteil 66 Infusionslösung vorliegen. Wird nunmehr die Balgpumpe 28 am unteren Totpunkt aus dem komprimierten Zustand in den expandierenden Zustand überführt, so werden wiederum die Klemmen 46, 74 und 148 geöffnet, während die Klemmen 48, 50, 58 und 154 geschlossen werden. Durch diesen Vorgang wird die mit Ultrafiltrat gefüllte Kammer 64 entleert, wobei zugleich Infusionslösung aus dem Infusionsbeutel oder -behälter 146 über die Leitung 144 in den Kammerteil 66 nachströmt und diesen füllt.

Wenn die Pumpe den oberen Totpunkt erreicht hat, werden die Klemmen 46, 74 und 148 geschlossen und es werden die Klemmen 48, 50, 58 und 154 geöffnet.

Das in den Kammerteil 64 einströmende Ultrafiltrat verdrängt dabei die im Kammerteil 66 befindliche Infusionslösung über die Leitung 150 in den Luftabscheider 42, d.h. es wird eine der Ultrafiltratmenge äquivalente Infusionslösungsmenge wiederum dem Blut zugesetzt, so daß es zu keinem Zeitpunkt zu einer Überwässerung des Patienten kommen kann. Dies ist insbesondere bei der Zuführung von hochkalorischen Lösungen ein erheblicher technischer Fortschritt.

Das Füllprogramm für den Infusionslösungsbereich ist nicht kritisch, da die in der Leitung befindliche Luft über den Luftabscheider entfernt werden kann. Üblicherweise wird man die Klemmen 148 und 154 beim Start- und Entleerungsprogramm vorteilhafterweise schließen.

Weiterhin ist auch dieses System als Disposable-Schlauchsystem in einem Stück steril erhältlich, so daß nur noch das Leitungsstück 144 an den Infusionslösungsbehälter 146 angeschlossen werden muß.

Um das gesamte Schlauchsystem von der Handhabung her besonders einfach zu gestalten, wird dieses, ggf. unter Einbeziehung des Filters 32, in einer in Fig. 1 und 5 gestrichelt mit 158 gezeigten Kassette, vorteilhafterweise aus einem Kunststoffmaterial, vereinigt, aus der lediglich die drei Schlauchanschlüsse 18, 44 und 70 gem. Fig. 1 bzw. vier Schlauchanschlüsse 18, 44, 74, 144 herausragen. Diese Kassette 158 läßt sich einfach in die Vorrichtung 10 einlegen und ist dann im eingelegten Zustand betriebsbereit. Vorteilhafterweise sind die Vorrichtung 10 und die Kassette 158 so konstruiert, daß sämtliche funktionsmäßig hergestellten Verbindungen zur Balgpumpe 28 bzw. zu den Absperrklemmen 46, 48, 50, 58, 74, 148, 154, sowie zu den Drosselorganen 52 bzw. 142 durch den Einlegevorgang hergestellt werden. Dies wird durch entsprechende, nicht gezeigte Ausnehmungen auf der Unterseite der Kassette 158 erreicht.

**Patentansprüche**

1. Vorrichtung zum Entfernen von Wasser aus Blut im extrakorporalen Kreislauf, umfassend ein Filter (32) zur Wasserabscheidung, das bluteingangsseitig mit einem ersten und einem zweiten Leitungsstück (18, 30), und blutausgangsseitig mit einer Ableitung (40, 44) verbunden ist und welches einen Filtratanschluß (38) zur Wasserabführung aufweist, wenigstens einen Blutanschluß (12), der über das erste und zweite Leitungsstück (18, 30) und über die Ableitung (40, 44) mit dem Filter (32) verbunden ist, eine Blutpumpe (28), welche von einem Überwachungs- und Steuergerät (126) steuerbar ist, und wenigstens eine Ultrafiltratmeßkammer (56), welche ein Innenvolumen $V_2$ aufweist, welche an ihrem Einlaß über ein drittes Leitungsstück (54) mit dem Filtratanschluß (38) verbunden ist und welche an ihrem Auslaß mit einem vierten Leitungsstück (70) verbunden ist, in welches eine fünfte Klemme (74) eingeschaltet ist, die mit dem Überwachungs- und Steuergerät (126) verbunden ist, dadurch gekennzeichnet,

daß die Blutpumpe (28) eine Pumpenkammer (20) beaufschlagt, die zwischen dem ersten Leitungsstück (18) und dem zweiten Leitungsstück (30) angeordnet ist und ein Innenvolumen $V_1$ aufweist,

daß in dem ersten Leitungsstück (18) eine erste Klemme (46) und in dem zweiten Leitungsstück (30) eine zweite Klemme (48) angeordnet sind,

daß in der Ableitung (40, 44) ein Absperrorgan (50) und ein Drosselorgan (52, 142) vorgesehen sind,

daß die erste Klemme (46) und das Absperrorgan (50) zusammen mit der zweiten Klemme (48) alternierend öffnen und schließen,

daß in das dritte Leitungsstück (54) eine vierte Klemme (58) eingeschaltet ist, die mit dem Überwachungs- und Steuergerät (126) verbunden ist,

daß das Überwachungs- und Steuergerät (126) die vierte und fünfte Klemme (58, 74) im Gegentakt steuert, den Takt dieser Klemmen (58, 74) überwacht und die Zahl der Taktzyklen speichert,

und daß das Verhältnis des Innenvolumens $V_1$ der Pumpkammer (20) zum Innenvolumen $V_2$ der Ultrafiltratmeßkammer (56) in einem Bereich von 5 : 1 bis 2 : 1 liegt.

2. Vorrichtung nach Anspruch 1, dadurch gekennzeichnet, daß das vierte Leitungsstück (70) mit einem Auffangbeutel (72) verbunden ist.

3. Vorrichtung nach Anspruch 1 oder 2, dadurch gekennzeichnet, daß die Ultrafiltratmeßkammer (56) durch eine elastische, erweiterbare Wand (68) in einen mit dem Filter (32) und dem Auffangbeutel (72) in Strömungsverbindung stehenden ersten Kammerteil (64) und einen zweiten Kammerteil (66) geteilt ist, der durch einen Stutzen (100, 106) zu belüften ist.

4. Vorrichtung nach Anspruch 1, dadurch gekennzeichnet, daß das Volumenverhältnis $V_1/V_2$ 3 : 1 beträgt.

5. Vorrichtung nach einem der Ansprüche 1 - 4, dadurch gekennzeichnet, daß das dritte Leitungsstück (54) eine hydrophobe, belüftbare Membran (62) aufweist.

6. Vorrichtung nach einem der Ansprüche 1 - 5, dadurch gekennzeichnet, daß das in der Ableitung (40, 44) angeordnete Absperrorgan (50) eine dritte Klemme (50) ist und daß die erste Klemme (46) und die fünfte Klemme (74) synchron zueinander und im Gegentakt zu der zweiten (48) und dritten Klemme (50) sowie der vierten Klemme (58) am unteren und oberen Totpunkt der Blutpumpe (28) umgeschaltet werden.

7. Vorrichtung nach einem der Ansprüche 1 - 6, dadurch gekennzeichnet, daß die Blutpumpe (28) einen Drucksensor (120), ein Unterdruckventil (122) und ein Überdruckventil (124) aufweist und daß, der Drucksensor (120), das Unterdruckventil (122) und das Überdruckventil (124) mit dem Überwachungs- und Steuergerät (126) verbunden sind.

8. Vorrichtung nach einem der Ansprüche 1 - 7, dadurch gekennzeichnet, daß in der vom Filter (32) abgehenden Leitung (40, 44) ein Luftabscheider (42) vorgesehen ist, der über ein fünftes Leitungsstück (150) mit dem zweiten Kammerteil (66) der Ultrafiltratmeßkammer (56) verbunden ist, der seinerseits über ein sechstes Leitungsstück (144) mit einem eine Infusionslösung aufweisenden Gefäß (146) verbunden ist.

9. Vorrichtung nach Anspruch 8, dadurch gekennzeichnet, daß im sechsten Leitungsstück (144) eine sechste Klemme (148) und im fünften Leitungsstück (150) eine siebte Klemme (154) vorgesehen sind, wobei die sechste Klemme (148) synchron zur ersten Klemme (46) und zur fünften Klemme (74) und im Gegentakt zur zweiten, dritten, vierten und siebten Klemme (48, 50, 58 und 154) gesteuert wird.

10. Vorrichtung nach einem der Ansprüche 1 - 9, dadurch gekennzeichnet, daß das erste (18), das zweite (30), das dritte (54) und das vierte Leitungsstück (70) sowie die Ableitung (40, 44) zu einem Schlauchsystem zusammengefaßt sind, das zusammen mit dem Filter (32) und der Ultrafiltratmeßkammer (56) in einer Kassette (158) untergebracht ist, wobei lediglich die Schlauchenden des ersten und vierten Leitungßstückes (18, 70) sowie das der Ableitung (44) aus der Kassette (158) ragen.

11. Vorrichtung nach Anspruch 10, dadurch gekennzeichnet, daß die Kassette (158) das fünfte und sechste Leitungsstück (150, 144) aufweist, wobei das Ende des sechsten Leitungsstücks (144) aus der Kassette (158) ragt.

12. Vorrichtung nach Anspruch 1, gekennzeichnet durch einen arteriellen Blutanschluß und einen venösen Blutanschluß.

**Revendications**

1. Appareil pour éliminer l'eau du sang dans un circuit extérieur à l'organisme, comprenant un filtre (32) de séparation de l'eau, qui communique, du côté de l'entrée du sang, avec un premier et un deuxième conduits (18, 30) et, du côté de la sortie du sang, avec un conduit de sortie (40, 44) et qui comprend un raccord pour le filtrat (38) destiné à l'évacuation de l'eau, au moins un raccord pour du sang (12), qui communique avec le filtre (32) par le premier et le deuxième conduits (18, 30) et par le conduit de sortie (40, 44), une pompe à sang (28), qui peut être commandée par un appareil de surveillance et de commande (126), et au moins une chambre de mesure de l'ultrafiltrat (56), qui a un volume intérieur $V_2$, dont l'entrée communique avec le raccord pour le filtrat (38) par un troisième conduit (54) et dont la sortie communique avec un quatrième conduit (70) dans lequel est montée une cinquième pince (74) qui est reliée a l'appareil de surveillance de commande (126), caractérisé

en ce que la pompe à sang alimente une chambre de pompage (20), qui est disposée entre le premier conduit (18) et le deuxième conduit (30) et qui a un volume intérieur $V_1$, en ce qu'une première pince (46) est montée dans le premier conduit (18) et une deuxième pince (48) dans le deuxième conduit (30), en ce que dans le conduit de sortie (40, 44) sont montés un organe d'arrêt (50) et un organe d'étranglement (52, 142), en ce que la première pince (46) et l'organe d'arrêt (50) s'ouvrent et se ferment alternativement en même temps que la deuxième pince (48), en ce qu'une quatrième pince (58) est montée dans le troisième conduit (54) et est reliée à l'appareil de surveillance et de commande (126), en ce que l'appareil de surveillance et de commande (126) commande les quatrième et cinquième pinces (58, 74) en opposition, surveille la cadence de fonctionnement de ces pinces (58, 74) et mémorise le nombre des cycles de fonctionnement, et en ce que le rapport du volume intérieur $V_1$ de la chambre de pompage (20) au volume intérieur $V_2$ de la chambre de mesure de l'ultrafiltrat (56) est compris entre 5 : 1 et 2 : 1.

2. Appareil suivant la revendication 1, caractérisé en ce que le quatrième conduit (70) communique avec une poche de réception (72).

3. Appareil suivant la revendication 1 ou 2, caractérisé en ce que la chambre de mesure de l'ultrafiltrat (56) est subdivisée par une paroi extensible (68) élastique, en une première partie de chambre (64) communiquant avec le filtre (32) et avec la poche de réception (72), et en une seconde partie de chambre (66) qui peut être mise à l'air libre par un raccord (100, 106).

4. Appareil suivant la revendication 1, caractérisé en ce que le rapport des volumes $V_1/V_2$ est de 3 : 1.

5. Appareil suivant l'une des revendications 1 à 4, caractérisé en ce que le troisième conduit (54) comporte une membrane (62) hydrophobe et perméable à l'air.

6. Appareil suivant l'une des revendications 1 à 5, caractérisé en ce que l'organe d'arrêt (50) disposé dans le conduit de sortie (40, 44), est une troisième pince (50), et en ce que la première pince (46) et la cinquième pince (74) sont serrées en synchronisme entre elles et en opposition avec la seconde pince (48) et la troisième pince (50), ainsi qu'avec la quatrième pince (58) aux points morts bas et haut de la pompe à sang (28).

7. Appareil suivant l'une des revendications 1 à 6, caractérisé en ce que la pompe à sang (28) comprend un détecteur de pression (120), une soupape de dépression (22) et une soupape de surpression (124), et en ce que le détecteur de pression (120), la soupape de dépression (122) et la soupape de surpression (124) sont reliés à l'appareil de surveillance et de commande (126).

8. Appareil suivant l'une des revendications 1 à 7, caractérisé en ce que, dans le conduit (40, 44) partant du filtre (32), est prévu un séparateur d'air (42) qui communique, par un cinquième conduit (150), avec la seconde partie de chambre (66) de la chambre de mesure de l'ultrafiltrat (56), laquelle communique à son tour, par un sixième conduit (144), avec un vase (146) contenant une solution de perfusion.

9. Appareil suivant la revendication 8, caractérisé en ce que, dans le sixième conduit (144), est prévue une sixième pince (148) et, dans le cinquième conduit (150), une septième pince (154), la sixième pince (148) étant commandée en synchronisme, par rapport à la première pince (46) et par rapport à la cinquième pince (74), et en opposition par rapport aux deuxième, troisième, quatrième et septième pinces (48, 50, 58 et 154)

10. Appareil suivant l'une des revendications 1 à 9, caractérisé en ce que le premier conduit (18), le deuxième conduit (30), le troisième conduit (54) et le quatrième conduit (70), ainsi que le conduit de sortie (40, 44) sont rassemblés en un système de conduits souples qui, ensemble avec le filtre (32) et la chambre de mesure de l'ultrafiltrat (56), est logé dans une cassette (158), seules les extrémités du premier et du quatrième conduits (18, 70), ainsi que celles du conduit de sortie (44) sortant de la cassette (158).

11. Appareil suivant la revendication 10, caractérisé en ce que la cassette (158) contient le cinquième et le sixième conduits (150, 144), l'extrémité du sixième conduit (144) sortant de la cassette (158).

12. Appareil suivant la revendication 1, caractérisé par un raccord pour le sang artériel et par un raccord pour le sang veineux.


## Claims

1. Apparatus for removing water from blood in an extra-corporeal circulation, comprises a filter (32) for water separation which is connected on the blood inlet side to a first and a second tube (18, 30) and on the blood outlet side to an offtake line (40, 44) and which has a filtrate connection (38) for water removal, at least one blood connection (12) which is connected via the first and second tube (18, 30) and via the offtake line (40, 44) to the filter (32), a blood pump (28) which is controllable by a monitoring and control device (126) and has at least one ultrafiltrate measuring chamber (56) having an inner volume $V_2$ and connected at its inlet via a third tube (54) to the filtrate connection (38) and connected at its outlet to a fourth tube (70) into which a fifth clamp (74) is inserted which is connected to the monitoring and control device (126), characterized in that the blood pump (28) acts on a pump chamber (20) which is disposed between the first tube (18) and the second tube (30) and has an inner volume $V_1$, that in the first tube (18) a first clamp (46) is disposed and in the second tube (30) a second clamp (48), that in the offtake line (40, 44) a shutoff member (50) and a throttle member (52, 142) are provided, that the first clamp (46) and the shutoff member (50) open and close alternately together with the second clamp (48), that in the third tube (54) a fourth clamp (58) is inserted which is connected to the monitoring and control device (126), that the monitoring and control device (126) controls the fourth and fifth clamp (58, 74) in opposite phase, the phase of said clamps (58, 74) being monitored and the number of cycles stored, and that the ratio of the inner volume $V_1$ of the pump chamber (20) to the inner volume $V_2$ of the ultrafiltrate measuring chamber (56) lies in a range from 5 : 1 to 2 : 1.

2. Apparatus according to claim 1, characterized in that the fourth tube (70) is connected to a collection bag (72).

3. Apparatus according to claim 1 or 2, characterized in that the ultrafiltrate measuring chamber (56) is divided by an elastic expandable wall (68) into a first chamber portion (64) in flow communication with the filter (32) and the collection bag (72) and a second chamber portion (66) which is to be vented through a connecting piece (100, 106).

4. Apparatus according to claim 1, characterized in that the volume ratio $V_1/V_2$ is 3 : 1.

5. Apparatus according to any one of claims 1 to 4, characterized in that the third tube (54) comprises a hydrophobic ventilatable membrane (62).

6. Apparatus according to any one of claims 1 to 5, characterized in that the shutoff member (50) disposed in the offtake line (40, 44) is a third clamp (50) and that the first clamp (46) and the fifth clamp (74) are switched synchronously with each other and in counterphase to the second (48) and third clamp (50) and the fourth clamp (58) at the bottom and top dead centre of the blood pump (28).

7. Apparatus according to any one of claims 1 to 6, characterized in that the blood pump (28) comprises a pressure sensor (120), a vacuum valve (122) and an excess pressure valve (124) and that the pressure sensor (120), the vacuum valve (122) and the excess pressure valve (124) are connected to the monitoring and control device (126).

8. Apparatus according to any one of claims 1 to 7, characterized in that in the line (40, 44) leading from the filter (32) an air separator (42) is provided which is connected via a fifth tube (150) to the second chamber (66) of the ultrafiltrate measuring chamber (56) which in turn is connected via a sixth tube (144) to a vessel (146) containing an infusion solution.

9. Apparatus according to claim 8, characterized in that in the sixth tube (144) a sixth clamp (148) is disposed and in the fifth tube (150) a seventh clamp (154), the sixth clamp (148) being controlled synchronously with the first clamp (46) and the fifth clamp (74) and in counterphase to the second, third, fourth and seventh clamp (48, 50, 58 and 154).

10. Apparatus according to any of claims 1 to 9, characterized in that the first (18), the second (30), the third (54) and the fourth tube (70) as well as the offtake line (40, 44) are combined to a tube system which together with the filter (32) and the ultrafiltrate measuring chamber (56) are assembled within a cassette (158) whereby merely the tube ends of the first and fourth tube (18, 70) as well as that of the offtake line (44) project out of the cassette (158).

11. Apparatus according to claim 10, characterized in that the cassette (158) comprises the fifth and sixth tube (150, 144) whereby the end of the sixth tube (144) projects out of the cassette (158).

12. Apparatus according to claim 1, characterized by an arterial blood connection and a venous blood connection.

FIG. 1

0 115 835

FIG. 2

18, 54

82    86         90

78

94    76

20,56

24,
68

102    98

106

110

26,    22,
66     64

104    108

100

112

96

80

88  84

92

30,70

3

FIG. 3

FIG. 5